# EUROPEAN PATENT SPECIFICATION

(11) **EP 1 053 221 B1**
(45) Date of publication and mention of the grant of the patent: **18.12.2002**
(21) Application number: 99904847.3
(22) Date of filing: 02.02.1999
(51) Int. Cl.: C07C 67/03, C07C 69/533, A61K 7/48

(54) **LIPID MIXTURES AND THEIR USE**
LIPIDMISCHUNG SOWIE IHRE VERWENDUNG
MELANGES LIPIDIQUES ET LEUR UTILISATION

(30) Priority: 04.02.1998 DE 19804376
(43) Date of publication of application: 22.11.2000
(73) Proprietor: JOHNSON & JOHNSON GmbH, 40474 Düsseldorf (DE)
(72) Inventor: FERNANDEZ-KLEINLEIN, Elena, D-53111 Bonn (DE); HAUSER, Matthias, D-53639 Koenigswinter (DE); VON STETTEN, Otto, D-52072 Aachen (DE)
(74) Representative: Groening, Hans Wilhelm, Dipl.-Ing.
(86) International application number: EP9900685
(87) International publication number: WO99039682

(56) References cited:
- EP-A- 0 014 308
- EP-A- 0 092 076
- EP-A- 0 432 835
- EP-A- 0 624 363

## Description

The invention relates to lipid mixtures having a physiologically effective amount of polyunsaturated fatty acids and/or derivatives thereof and also to their use as such or in cosmetic or pharmaceutical preparations for topical application to the skin.

The principal task of topically applied lipids, in the cosmetic or pharmaceutical field, is to enhance the barrier function, i.e. the protection function, of the skin and thus to prevent it from drying out or to prevent the entry of foreign substances. They also serve to improve the suppleness of the skin, the condition of its surface and its appearance. Moreover, substances present particularly in natural lipids, e.g. saturated and unsaturated fatty acids, lecithins, ceramides, etc., play an important role in physiological processes, e.g. metabolism, irritation, inflammation, allergic reactions, etc.

The barrier function is often enhanced by using occlusive substances, e.g. paraffin oil and vaseline, which, although preventing the skin from drying out to a certain degree, are not, however, able to contribute to the physiological processes in the skin. Furthermore, their use is cosmetically unacceptable since they have poor spreadability and poor absorption and impart a greasy feel to the skin.

Natural lipids are able, depending on the composition, to influence skin physiology, but they are more difficult to spread than the specified occlusive substances, cannot be distributed particularly well and are absorbed more slowly than, for example, paraffin oil.

US-A-5,445,822 describes a cosmetic composition comprising triglycerides containing from 40 to 70% of oleic acid, from 30 to 50% of polyunsaturated fatty acids, from 0.2 to 1% of γ-linolenic acid and from 1 to 5% of α-linolenic fatty acid, the ratio of n-6 fatty acids to n-3 fatty acids being from 10:1 to 30:1. There is no mention of the application properties, nor is there a further explanation as to the physiological effects of the composition.

The object of the present invention is to provide lipid mixtures which contribute to improving the aforementioned application properties at the same time as improving the physiological condition of the skin.

According to the invention, the object is achieved by a generic lipid mixture having an effective content of modified coconut oil, which essentially comprises C10 - C14 fatty acids in the form of mono-, di-and triglycerides and has a cloud point of < 5°C.

Preference is given to a lipid mixture in which the modified coconut oil is present in a concentration of from 5 to 40%, preferably from 10 to 30%, particularly preferably from 15 to 25%, based on the total weight of the lipid mixture.

The polyunsaturated fatty acids advantageously comprise linoleic acid and α-linolenic acid.

It is intended that the ratio of linoleic acid to α-linolenic acid, in percent by weight, is between 15:1 and 5:1, preferably between 13:1 and 7:1, particularly preferably between 11:1 and 9:1.

It is intended that the polyunsaturated fatty acids are essentially present in the form of their triglycerides.

Natural triglycerides having a high content of polyunsaturated fatty acids are advantageously present.

According to a particular embodiment of the invention, the polyunsaturated fatty acids are prepared using soybean oil, sunflower oil, wheat germ oil, linseed oil, thistle oil, China oil, grapeseed oil, sesame oil or mixtures thereof.

It is advantageous to use a mixture of at least two of the specified oils.

The invention further relates to the use of the lipid mixture according to the invention as such or in a cosmetic preparation.

Finally, the invention also relates to the use of the lipid mixture according to the invention as or in a pharmaceutical preparation for topical application to the skin, in particular for the treatment of dry skin, neurodermatitis, atopic eczema and similar skin conditions.

Surprisingly, it has been found that the lipid mixture according to the invention has better application properties than the known occlusive substances, such as, for example, paraffin oil and vaseline, or individual natural oils, and at the same time contributes to improving the physiological condition of the skin.

An essential component of the lipid mixture according to the invention is a modified coconut oil, which essentially comprises C10-C14 fatty acids in the form of mono-, di- and triglycerides, and has a cloud point of < 5°C. A modified coconut oil of this type is known under the INCI name "cocoglycerides" and is commercially available, for example under the name Myritol 331.

It is advantageously used in a concentration of from 5 to 40%, preferably from 10 to 30% and particularly preferably from 15 to 25%, based on the total weight of the lipid mixture. A direct comparison with the compositions in the aforementioned US-A-5,445,822 has shown that the triglycerides comprising oleic acid mentioned therein do not improve the application properties in this way. Moreover, the oxidation stability of the coconut oil used according to the invention is significantly higher than that of the triglycerides listed in the cited US Patent.

The physiologically effective components used are lipids having a high content of linoleic acid and α-linolenic acid, where the ratio of linoleic acid to α-linolenic acid, in percent by weight, is between 15:1 and 5:1, preferably between 13:1 and 7:1 and particularly preferably between 11:1 and 9:1. Linoleic acid and α-linolenic acid can either be used as such or in the form of their derivatives, e.g. monoesters, triglycerides or amides, etc.

Triglycerides having a high content of linoleic acid and α-linolenic acid are present in natural oils, such as, for example, soybean oil, sunflower oil, wheat germ oil, linseed oil, thistle oil, China oil, grapeseed oil or sesame oil. Particular preference is given to soybean oil, sunflower oil, wheat germ oil, linseed oil, thistle oil or China oil, with soybean oil, sunflower oil, wheat germ oil and linseed oil being the most preferred. In particular, it is possible to use mixtures of at least two of the specified oils. In addition, γ-linolenic acid can be added to the lipid mixture as it is or in the form of oils such as starflower oil or evening primrose oil.

It is preferable to add an antioxidant to the lipid mixtures. The antioxidants which are primarily suitable for this purpose are oil-soluble ones, such as, for example, tocopherol, mixtures of different tocopherols, ascorbyl palmitate, BHT, BHA and mixtures thereof, optionally with the addition of synergists, such as lecithin etc. Antioxidants of this type are commercially available, for example under the names Oxynex, Covi-ox T70 or Controx. Furthermore, it is possible to add other auxiliaries or additives, such as, for example, perfume or the like.

The lipid mixtures according to the invention are either used as such, e.g. as body oils for cosmetic or pharmaceutical use, or as oil phases or constituents of oil phases in cosmetic or pharmaceutical preparations, such as, for example, emulsions of the water-in-oil type or of the oil-in-water type, microemulsions, nonaqueous preparations in the form of pastes, sticks, ointments, oil baths, hydrosurfactant preparations such as care shampoo and shower gels, etc. Substances which are customarily used in the formulation of such preparations can be added to the corresponding cosmetic or pharmaceutical preparations. These include, inter alia, emulsifiers, surfactants, emollients, structure formers, etc. Furthermore, it is possible, depending on the desired use, to incorporate further active substances, e.g. light protection filters, antibacterial substances, antiperspirants, deodorants, etc.

The lipid mixtures according to the invention and the cosmetic or pharmaceutical preparations prepared therefrom are notable for excellent absorption, good spreadability and distribution properties and impart an exceptionally good and smooth feel to the skin at the same time as being very well tolerated. They are suitable, in particular, for use with dry skin, neurodermatitis, atropic eczema or similar skin conditions.

People who suffer from atropic eczema usually avoid oil- and fat-containing skincare products since such products worsen the condition of the skin rather than improve it. Surprisingly, it has now been found that the lipid mixtures according to the invention in the form of body oils or emulsions are not only very well tolerated by people with atopic eczema but that the disease phenomena can be reduced and the condition of the skin significantly improved. In an application test using a body oil according to the invention, participants who suffered from atopic eczema reported that the red areas and centres of inflammation disappeared within one week. People with dry skin reported a significant increase in the elasticity of the skin and a normalizing of the condition of the skin.

The invention is illustrated in more detail below with reference to typical lipid mixtures and products prepared therefrom. Examples 1 to 6 give lipid mixtures according to the invention which were prepared by thoroughly mixing, by stirring, the individual components in the order given in a stainless steel container.

### Example 1:

| | |
|---|---|
| Modified coconut oil | 15.00% by weight |
| Soybean oil | 43.17 % by weight |
| Wheat germ oil | 41.53 % by weight |

### Example 2:

| | |
|---|---|
| Modified coconut oil | 20.00 % by weight |
| Soybean oil | 2.51 % by weight |
| Sunflower oil | 71.89 % by weight |
| Oxynex K | 0.10 % by weight |

### Example 3:

| | |
|---|---|
| Modified coconut oil | 10.39 % by weight |
| Sunflower oil | 71.34 % by weight |
| Linseed oil | 6.87 % by weight |
| Thistle oil | 11.20 % by weight |
| Oxynex K | 0.10% by weight |
| Perfume | 0.10 % by weight |

### Example 4:

| | |
|---|---|
| Modified coconut oil | 19.89 % by weight |
| Soybean oil | 56.96 % by weight |
| Sunflower oil | 12.86 % by weight |
| Wheat germ oil | 10.00 % by weight |
| Oxynex K | 0.15 % by weight |
| Perfume | 0.15 % by weight |

### Example 5:

| | |
|---|---|
| Modified coconut oil | 19.86 % by weight |
| Soybean oil | 24.86 % by weight |
| Sunflower oil | 50.08 % by weight |
| Linseed oil | 5.00 % by weight |
| Oxynex K | 0.10 % by weight |
| Perfume | 0.10 % by weight |

### Example 6:

| | |
|---|---|
| Modified coconut oil | 20.02 % by weight |
| Soybean oil | 59.10% by weight |
| Sunflower oil | 16.68 % by weight |
| Wheat germ oil | 4.00 % by weight |
| Oxynex K | 0.10 % by weight |
| Perfume | 0.10 % by weight |

### Example 7:

An oil-in-water emulsion was prepared by incorporating the lipid mixture from Example 4 into a solution of Pemulen TR1 in water and propylene glycol. After the other ingredients, which are given the table below, had been added, the mixture was neutralized using sodium hydroxide solution and homogenized.

| | |
|---|---|
| Lipid mixture from Ex. 4 | 46.60 % by weight |
| Tocopheryl acetate | 0.10 % by weight |
| Preservative | 1.00 % by weight |
| Pemulen TR1 | 0.35 % by weight |
| Arlacel 83 | 0.10 % by weight |
| Water | 50.35 % by weight |
| Propylene glycol | 1.00 % by weight |
| Na hydroxide | 0.50 % by weight |

An oil according to Example 4 was tested by a panel of experts for its application properties compared with paraffin oil and individual natural oils. The properties of spreading and distribution were judged to be significantly better compared with the individual natural oils; the properties of skin smoothness and absorption were significantly better compared with paraffin oil and the individual natural oils.

The features of the invention disclosed in the description above and the claims may be essential either individually or in any combination for implementing the invention in its different variants.

## Claims

1. Lipid mixture having a physiologically effective content of polyunsaturated fatty acids and/or derivatives thereof, **characterized by** an effective content of modified coconut oil which essentially comprises C10 to C14 fatty acids in the form of mono-, di- and triglycerides and has a cloud point of < 5°C.

2. Lipid mixture according to Claim 1, **characterized in that** the modified coconut oil is present in a concentration of from 5 to 40%, based on the total weight of the lipid mixture.

3. Lipid mixture according to Claim 2, **characterized in that** the modified coconut oil is present in a concentration of from 10 to 30%, based on the total weight of the lipid mixture.

4. Lipid mixture according to Claim 3, **characterized in that** the modified coconut oil is present in a concentration of from 15 to 25%, based on the total weight of the lipid mixture.

5. Lipid mixture according to Claim 1, **characterized in that** the polyunsaturated fatty acids are linoleic acid and α-linolenic acid.

6. Lipid mixture according to Claim 5, **characterized in that** the ratio of linoleic acid to α-linolenic acid, in percent by weight, is between 15:1 and 5:1.

7. Lipid mixture according to Claim 6, **characterized in that** the ratio of linoleic acid to α-linolenic acid, in percent by weight, is between 13:1 and 7:1.

8. Lipid mixture according to Claim 7, **characterized in that** the ratio of linoleic acid to α-linolenic acid is between 11:1 and 9:1.

9. Lipid mixture according to one of Claims 4 to 8, **characterized in that** the Polyunsaturated fatty acids are essentially in the form of their triglycerides.

10. Lipid mixture according to Claim 9, **characterized in that** natural triglycerides having a high content of polyunsaturated fatty acids are present.

11. Lipid mixture according to Claim 10, **characterized in that** polyunsaturated fatty acids are prepared using soybean oil, sunflower oil, wheat germ oil, linseed oil, thistle oil, China oil, grapeseed oil, sesame oil of mixtures thereof.

12. Lipid mixture according to Claim 11, **characterized in that** a mixture of at least two of the specified oils is used.

13. Use of a lipid mixture according to one of the preceding claims for the manufacture of cosmetic preparation.

14. Use of a lipid mixture according to one of Claims 1 to 12 for the manufacture of a medicament for topical application to the skin.

15. Use according to Claim 14 for the manufacture of a medicament for the treatment of dry skin, neurodermatitis, atopic eczema and similar skin conditions.

## Patentansprüche

1. Lipidmischung mit einem physiologisch wirksamen Anteil an mehrfach ungesättigten Fettsäuren und/oder deren Derivaten, **gekennzeichnet durch** einen wirksamen Gehalt an modifiziertem Kokosöl, das im wesentlichen C10- bis C14-Fettsäuren in Form von Mono-, Di- und Triglyceriden enthält und einen Trübungspunkt von < 5°C aufweist.

2. Lipidmischung nach Anspruch 1, **dadurch gekennzeichnet, daß** das modifizierte Kokosöl in einer Konzentration von 5 bis 40%, bezogen auf das Gesamtgewicht der Lipidmischung, enthalten ist.

3. Lipidmischung nach Anspruch 2, **dadurch gekennzeichnet, daß** das modifizierte Kokosöl in einer Konzentration von 10 bis 30%, bezogen auf das Gesamtgewicht der Lipidmischung, enthalten ist.

4. Lipidmischung nach Anspruch 3, **dadurch gekennzeichnet, daß** das modifizierte Kokosöl in einer Konzentration von 15 bis 25%, bezogen auf das Gesamtgewicht der Lipidmischung, enthalten ist.

5. Lipidmischung nach Anspruch 1, **dadurch gekennzeichnet, daß** die mehrfach ungesättigten Fettsäuren Linolsäure und α-Linolensäure sind.

6. Lipidmischung nach Anspruch 5, **dadurch gekennzeichnet, daß** das Verhältnis von Linolsäure zu α-Linolensäure in Gewichtsprozenten zwischen 15:1 und 5:1 liegt.

7. Lipidmischung nach Anspruch 6, **dadurch gekennzeichnet, daß** das Verhältnis von Linolsäure zu α-Linolensäure in Gewichtsprozenten zwischen 13:1 und 7:1 liegt.

8. Lipidmischung nach Anspruch 7, **dadurch gekennzeichnet, daß** das Verhältnis von Linolsäure zu α-Linolensäure zwischen 11:1 und 9:1 liegt.

9. Lipidmischung nach einem der Ansprüche 4 bis 8, **dadurch gekennzeichnet, daß** die mehrfach ungesättigten Fettsäuren im wesentlichen in Form ihrer Triglyceride vorliegen.

10. Lipidmischung nach Anspruch 9, **dadurch gekennzeichnet, daß** natürliche Triglyceride mit einem hohen Gehalt an mehrfach ungesättigten Fettsäuren enthalten sind.

11. Lipidmischung nach Anspruch 10, **dadurch gekennzeichnet, daß** zur Bereitstellung der mehrfach ungesättigten Fettsäuren Sojaöl, Sonnenblumenöl, Weizenkeimöl, Leinöl, Distelöl, Chinaöl, Traubenkernöl, Sesamöl oder Mischungen derselben eingesetzt werden.

12. Lipidmischung nach Anspruch 11, **dadurch gekennzeichnet, daß** eine Mischung von wenigstens zwei der genannten Öle eingesetzt wird.

13. Verwendung einer Lipidmischung nach einem der vorangehenden Ansprüche zur Herstellung einer kosmetischen Zubereitung.

14. Verwendung einer Lipidmischung nach einem der Ansprüche 1 bis 12 zur Herstellung eines Medikaments zur topischen Applikation auf der Haut.

15. Verwendung nach Anspruch 14 zur Herstellung eines Medikaments für die Behandlung von trockener Haut, Neurodermitits, atopischem Ekzem und ähnlichen Hautzuständen.

## Revendications

1. Mélange de lipides ayant une teneur physiologiquement efficace en acides gras poly-insaturés et/ou en dérivés de ceux-ci, **caractérisé par** une teneur efficace en huile de noix de coco modifiée qui comprend essentiellement des acides gras en C₁₀ à C₁₄ sous forme de mono-, di- et triglycérides et qui a un point de trouble de < 5 °C.

2. Mélange de lipides selon la revendication 1, **caractérisé en ce que** l'huile de noix de coco modifiée est présente à une concentration de 5 à 40 %, par rapport au poids total du mélange de lipides.

3. Mélange de lipides selon la revendication 2, **caractérisé en ce que** l'huile de noix de coco modifiée est présente à une concentration de 10 à 30 %, par rapport au poids total du mélange de lipides.

4. Mélange de lipides selon la revendication 3, **caractérisé en ce que** l'huile de noix de coco modifiée est présente à une concentration de 15 à 25 %, par rapport au poids totale du mélange de lipides.

5. Mélange de lipides selon la revendication 1, **caractérisé en ce que** les acides gras poly-insaturés sont l'acide linoléique et l'acide α-linolénique.

6. Mélange de lipides selon la revendication 5, **caractérisé en ce que** le rapport de l'acide linoléique à l'acide α-linolénique, en pourcentage pondéral, est entre 15/1 et 5/1.

7. Mélange de lipides selon la revendication 6, **caractérisé en ce que** le rapport de l'acide linoléique à l'acide α-linolénique, en pourcentage pondéral, est entre 13/1 et 7/1.

8. Mélange de lipides selon la revendication 7, **caractérisé en ce que** le rapport de l'acide linoléique à l'acide α-linolénique est entre 11/1 et 9/1.

9. Mélange de lipides selon l'unc des revendications 4 à 8, **caractérisé en ce que** les acides gras poly-insaturés sont essentiellement sous forme de leurs triglycérides.

10. Mélange de lipides selon la revendication 9, **caractérisé en ce que** des triglycérides naturels ayant une teneur élevée en acides gras poly-insaturés sont présents.

11. Mélange de lipides selon la revendication 10, **caractérisé en ce que** les acides gras poly-insaturés sont préparés en utilisant de l'huile de soja, de l'huile de tournesol, de l'huile de germe de blé, de l'huile de lin, de l'huile de chardon, de l'huile de bois de Chine, de l'huile de pépins de raisin, de l'huile de sésame ou des mélanges de celles-ci.

12. Mélange de lipides selon la revendication 11, **caractérisé en ce qu'**on utilise un mélange d'au moins deux des huiles spécifiées.

13. Utilisation d'un mélange de lipides selon l'une quelconque des revendications précédentes pour la fabrication d'une préparation cosmétique.

14. Utilisation d'un mélange de lipides selon l'une quelconque des revendications 1 à 12 pour la fabrication d'un médicament pour application topique sur la peau.

15. Utilisation selon la revendication 14 pour la fabrication d'un médicament pour le traitement, des peaux sèches, d'une névrodermite circonscrite, d'un eczéma constitutionnel et d'affections cutanées similaires.
